# EUROPEAN PATENT SPECIFICATION

(11) **EP 2 401 389 B1**
(45) Date of publication and mention of the grant of the patent: **14.04.2021**
(21) Application number: 10705867.9
(22) Date of filing: 25.02.2010
(51) Int. Cl.: C12P 19/44

(54) **SYNTHESIS OF LONG-CHAIN ALKYL GLYCOSIDES**
SYNTHESE LANGKETTIGER ALKYLGLYKOSIDE
SYNTHÈSE D'ALKYL GLYCOSIDES À CHAÎNE LONGUE

(30) Priority: 25.02.2009 US 208500 P
(43) Date of publication of application: 04.01.2012
(73) Proprietor: Enza Biotech AB, 221 00 Lund (SE)
(72) Inventor: Adlercreutz, Patrick, 224 60 Lund (SE); Svensson, David, 226 57 Lund (SE); Mathew, Sindhu, Trivandrum, Kerala 695018 (IN); Nordberg Karlsson, Eva Margareta, 227 38 Lund (SE)
(74) Representative: Craven, Ian
(86) International application number: PCT/EP2010/052367
(87) International publication number: WO 2010/097421

(56) References cited:
- SVENSSON DAVID ET AL: "Efficient Synthesis of a Long Carbohydrate Chain Alkyl Glycoside Catalyzed by Cyclodextrin Glycosyltransferase (CGTase)" BIOTECHNOLOGY AND BIOENGINEERING, vol. 104, no. 5, December 2009 (2009-12), pages 854-861, XP002592613 ISSN: 0006-3592
- SVENSSON, D ET AL.: "Enzymatic route to alkyl glycosides having oligomeric head groups" GREEN CHEMISTRY, vol. 11, 3 June 2009 (2009-06-03), pages 1222-1226, XP008124531
- YOON S H ET AL: "Optimized synthesis of specific sizes of maltodextrin glycosides by the coupling reactions of Bacillus macerans cyclomaltodextrin glucanyltransferase" CARBOHYDRATE RESEARCH, PERGAMON, GB LNKD- DOI:10.1016/J.CARRES.2005.11.014, vol. 341, no. 2, 6 February 2006 (2006-02-06), pages 210-217, XP025010563 ISSN: 0008-6215 [retrieved on 2006-02-06] cited in the application
- ZHAO HAISUO ET AL: "Cyclomaltodextrin Glucanotransferase-Catalyzed Transglycosylation from Dextrin to Alkanol Maltosides" BIOSCIENCE BIOTECHNOLOGY AND BIOCHEMISTRY, vol. 72, no. 11, 7 November 2008 (2008-11-07), pages 3006-3010, XP002592615 ISSN: 0916-8451 cited in the application
- DATABASE WPI Week 199833 Thomson Scientific, London, GB; AN 1998-379989 XP002592616 & JP 10 150986 A (IMANAKA T) 9 June 1998 (1998-06-09)
- VETTER D ET AL: "CHAIN LENGTH SPECIFICITY OF CYCLODEXTRIN GLYCOSYLTRANSFERASE" STARCH, vol. 44, no. 6, 1992, pages 229-233, XP002592617 ISSN: 0038-9056 cited in the application
- PARK DONG-CHAN ET AL: "Characteristics of transglycosylation reaction of cyclodextrin glucanotransferase in the heterogeneous enzyme reaction system using extrusion starch as a glucosyl donor" ENZYME AND MICROBIAL TECHNOLOGY, vol. 22, no. 4, March 1998 (1998-03), pages 217-222, XP002592618 ISSN: 0141-0229
- OKADA T ET AL: "Intermolecular transglycosylating reaction of cyclodextrin glucanotransferase immobilized on capillary membrane" JOURNAL OF FERMENTATION AND BIOENGINEERING, SOCIETY OF FERMENTATION TECHNOLOGY, JP LNKD- DOI:10.1016/0922-338X(94)90231-3, vol. 77, no. 3, 1 January 1994 (1994-01-01), pages 264-267, XP023573490 ISSN: 0922-338X [retrieved on 1994-01-01]
- QINGSHENG QI ET AL: "Cyclodextrin glucanotransferase: from gene to applications" APPLIED MICROBIOLOGY AND BIOTECHNOLOGY, SPRINGER, BERLIN, DE LNKD- DOI:10.1007/S00253-004-1781-5, vol. 66, no. 5, 1 February 2005 (2005-02-01), pages 475-485, XP019331746 ISSN: 1432-0614 cited in the application
- RICHARD G ET AL: "Glucosylation of alpha-butyl- and alpha-octyl-d-glucopyranosides by dextransucrase and alternansucrase from Leuconostoc mesenteroides" CARBOHYDRATE RESEARCH, PERGAMON, GB LNKD- DOI:10.1016/S0008-6215(03)00070-3, vol. 338, no. 9, 22 April 2003 (2003-04-22), pages 855-864, XP004418222 ISSN: 0008-6215 cited in the application

## Description

### BACKGROUND OF THE INVENTION

### Field of the Invention

The present invention relates generally to enzymatic synthesis of alkyl glycosides having oligomeric head groups using enzymes selected from the group of cyclomaltodextrin glucanotransferase (EC number 2.4.1.19) and 4-alpha-glucanotranserase, (EC number 2.4.1.25).

### Description of the Related Art

Surfactants have been used for many years in a plethora of pharmaceutical, cosmetic, personal care, household and industrial formulations because of their wide variety of applications, such as foaming, emulsification, dispersion, detergency, spreading and wetting.

Surfactants contain one hydrophobic part and one hydrophilic part. The hydrophobic part is often one or a couple of hydrocarbon chains. Concerning the hydrophilic part, there is a vast variety of structures that are possible. This part can be positively charged (cationic surfactants), negatively charged (anionic surfactants) or not charged (non-ionic surfactants).

For some of these applications there is a need for surfactants having both long hydrophobic parts and long hydrophilic parts. In many cases, such as in pharmaceuticals and personal care products, it is also required that the surfactant be nontoxic and non-irritating to skin and mucosa. Furthermore, the products should have acceptable stability and due to the increasing awareness of environmental effects, they should preferably be biodegradable.

OECD (Organization for Economic Cooperation and Development) has set up readily biodegradable screening tests (OECD 301) and in the Detergent Regulation (EC) No 648/2004 of the European Parliament and of the Council of 31 March 2004 biodegradability criteria on surfactants have been defined.

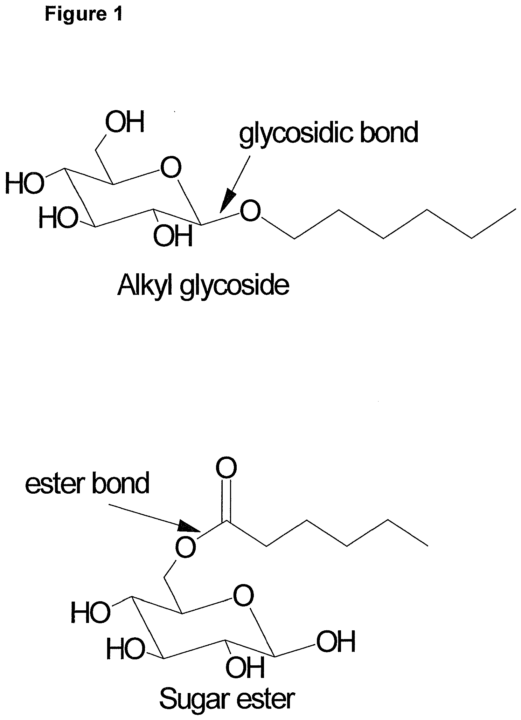

Alkyl glycosides are surfactants that match the criteria mentioned above quite well. The hydrophilic part is typically a monosaccharide residue for example, glucose (most frequently), fructose, mannose, galactose, arabinose, hexosas and sometimes sugars such as digitalose or cymarose; or a chain of monosaccharide residues joined by glycosidic linkages, and a long-chain alcohol is attached via another glycosidic linkage (Figure 1). Sugar esters constitute a similar group of surfactant molecules, but their stability is much poorer especially in alkaline media, due to the instability of the ester bond.

Complex mixtures of alkyl glycosides are commercially available under the name alkyl polyglycosides, APGs. These products are synthesised using either one-stage or two-stage synthesis. In both synthesis variants, the carbohydrate is suspended in an excess of alcohol (2-6 mol) and the reaction is carried out at a temperature above 100 °C in the presence of an acidic catalyst (usually a sulfonic acid) ^{[1]}. In the two-stage process the carbohydrate is first coupled to a short alcohol (usually butanol) producing an alkyl polyglycoside, which subsequently is used as a substrate in the second stage in which the short alcohol is exchanged with a longer one.

Alkyl polyglycosides contain a large number of different molecules differing in the number of monosaccharide residues and how they are bound to each other. Due to the relatively harsh reaction conditions used in prior art reactions and the non-selective acid catalyst, several side reactions occur and side products such as glucose polymers, ethers and coloured impurities are formed ^{[1]}. A main drawback of the APGs is thus the very high number of molecular species present, some of which can have unwanted effects.

Alkyl glycosides similar to those made in the present invention can also be prepared by chemical catalysis. In those methods, protection/deprotection reactions are needed. Typically, all hydroxyl groups in the oligosaccharide that are to be converted to alkyl glycoside are reacted with a reagent converting them to less reactive ester groups. A common procedure is to let the hydroxyl groups react with acetic anhydride to put acetyl groups on all the hydroxyls. Then the protected oligosaccharide reacts with an alcohol using a Lewis acid catalyst. Typical catalysts used are FeCl₃ ^{[2]} and SnCl₄ ^{[3]}. However, due to the low reactivity of the protected oligosaccharides, more active heteropolyacids, such as phosphotungstic acid or phosphomolybdic acid have been used as catalysts instead ^{[2]}. In any case, large amounts of catalyst (50-100 % by weight compared to the amount of oligosaccharide) must be used, the reaction temperature must be high (80 °C) and the yields are moderate (40-70%). Furthermore, the products contain a mixture of α and β anomers, even when a pure α anomer of the protected oligosaccharide is used as starting material ^{[2]}. After the reaction, the protecting groups must be removed in an additional step.

Drawbacks with this prior methodology include:
- The reactions involve several reaction steps including introduction and removal of protecting groups, such as acetylation/deacetylation of the hydroxyl groups of the carbohydrate part
- The reactions involve the use of aggressive reagents, such as acetic anhydride and organic solvents, such as toluene and methanol.
- Risks of side reactions due to the reactive catalyst
- Risks of remaining catalyst, such as FeCl₃ and SnCl₄ in the product
- Formation of both α and β anomers of the product. The procedure of Katsuraya et al generates mainly the β-anomer with 10-31 % of the α-anomer ^{[2]}. Different kinds of enzymes can be used as catalysts for surfactant synthesis. Lipases are used for synthesis of sugar esters and glycosyl hydrolases are used for the synthesis of alkyl glycosides. The most straightforward way to use glycosyl hydrolases for the synthesis of alkyl glycosides is to carry out a direct condensation of a carbohydrate and an alcohol. These reactions are hampered by the fact that the two substrates are poorly miscible and even with the addition of solvents it is very difficult to reach appreciable concentration of the two substrates in the same liquid phase. These problems increase in severity with increasing length of the carbohydrate and of the alcohol ^{[4]}. The achievable yields are thus low.

An attractive alternative is to first synthesize an alkyl glycoside with a moderate length of the carbohydrate part and subsequently elongate the carbohydrate part. Enzymes used for the elongation reaction include dextransucrase, which catalyzes 1-6 transfer reactions, and alternansucrase, which catalyzes 1-3 and 1-6 transfer reactions. These enzymes use sucrose as glycosyl donor, and the carbohydrate group of alkyl glycosides can be elongated with one glucose residue at a time ^{[5]}. With this approach, butyl- and octyl-α-D-glucopyranoside were extended, but rather complex product mixtures were obtained containing alkyl glycosides with up to 4 glucose residues. The Japanese company Hayashibara Biochemical Laboratories Inc has recently developed a new enzyme from *Bacillus stearothermophilus* which has been used for glycosylation of surfactants. The enzyme is called cyclodextrin glucanotransferase (CGTase) ^{[6, 7]}. Using this enzyme with sucrose laurate (a sugar ester) as acceptor, products with 1-3 additional glucose residues were formed ^{[6]}. As mentioned above the stability of such compounds is poor due to the presence of the ester bond. Acceptors with longer hydrocarbon chains were not tried in this study ^{[6]}. Also alkyl maltosides have been used as acceptors in glycosylation reactions catalyzed by this enzyme with dextrin as glycosyl donor ^{[7]}. Only one or two glucose residues were added to the acceptors thus producing products with maximum 4 glucose residues, The main problem with previously described methods to synthesize alkyl glycosides containing more than 7 monosaccharide residues is that they involve protection and deprotection steps ^{[3][2]}, which makes them time consuming and expensive. Furthermore, they involve risks of side reactions: formation of isomers and of other products due to the non-selective catalysts. In addition, they generate large amounts of waste.

Accordingly, there is a need for new and more efficient methods to prepare long-chain alkyl glycosides. Thus, it would be advantageous to provide a method for the synthesis of said long chain alkyl glycosides in which an enzyme is used as a catalyst under mild reaction conditions, so as to minimize side reactions and reduce the amounts of waste that are produced. It would further be advantageous to avoid the generation of anomer mixtures in said reactions. The present invention satisfies this need and provides related advantages as well.

### SUMMARY OF THE INVENTION

The present invention provides a process as defined in the claims.

The present invention puts forth the novel insight that long-chain alkyl glycosides can be synthesized by the method described herein in a safe and effective manner. The method has the advantages that it may be done in one step, no protection/deprotection reactions are needed and water may be used as solvent instead of organic solvents. To attain this, the present invention uses an enzyme as catalyst under mild reaction conditions, which minimizes side reactions and minimal amounts of waste are produced. Still another advantage compared to previously described methods is that no anomer mixtures are generated.

According to the invention herein, the enzyme is capable to transfer carbohydrate parts of at least 2, typically 6-9, but dependent on the enzyme used up to at least 26 monosaccharide residues from a glycosyl donor to a glycosyl acceptor, which should be an alkyl glycoside or a mixture of alkyl glycosides. The carbohydrate part of the acceptor is thus elongated The enzyme is selected from the group of cyclomaltodextrin glucanotransferase (EC number 2.4.1.19) and 4-alpha-glucanotranserase, (EC number 2.4.1.25).

The present invention makes it possible to synthesize alkyl glycosides with oligomeric head groups. The alkyl chains preferably contain at least 7 carbon atoms and the head groups preferably contain at least 3 monosaccharide residues, although product mixtures can contain molecules outside these specifications.

The alkyl glycosides of the present invention may have long hydrophobic and hydrophilic parts and include compounds of the general formula Gₘ₊ₓ-OR.

They are synthesized according to the following scheme:

Gₙ + Gₘ-OR → Gₙ₋ₓ + Gₘ₊ₓ-OR

wherein:
Gₙ is a glycosyl donor, G is a monosaccharide residue having 5 or 6 carbon atoms, n is the number of monosaccharide residues in the glycosyl donor, Gₘ-OR is a glycosyl acceptor, m is the number of monosaccharides residues of the acceptor, R is a monovalent radical or an alkyl group, and x is the number of residues that the enzyme transfers from the glycosyl donor to the glycosyl acceptor.

There has thus been outlined, rather broadly, the more important features of the invention in order that the detailed description thereof may be better understood, and in order that the present contribution to the art may be better appreciated. There are additional features of the invention that will be described hereinafter.

In this respect, before explaining at least one embodiment of the invention in detail, it is to be understood that the invention is not limited in its application to the details of construction and to the arrangements of the components set forth in the following description or illustrated in the drawings. The invention is capable of other embodiments and of being practiced and carried out in various ways. Also, it is to be understood that the phraseology and terminology employed herein are for the purpose of the description and should not be regarded as limiting.

### BRIEF DESCRIPTION OF THE DRAWINGS

Figure 1 depicts two important types of carbohydrate based surfactants: alkyl glycosides and sugar esters.

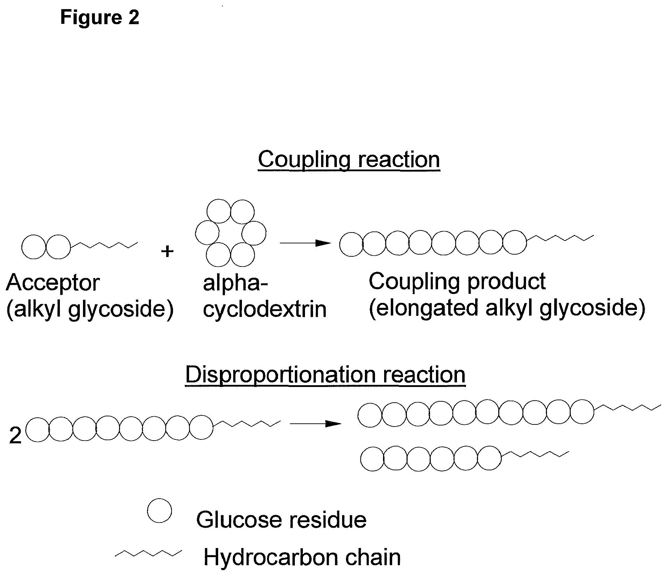

Figure 2 is a schematic view of the coupling and disproportionation reactions in the synthesis of alkyl glycosides having oligomeric head groups. The size of the cyclic carbohydrate can vary from 6 up to cyclic amyloses having up to at least 26 monosaccharide residues. Likewise, the size of the noncyclic substrates can vary from short oligosaccharides to long (or even branched) polysaccharides. These reactions are catalyzed by an enzyme, such as a cyclomaltodextrin glucanotransferase (CGTase) or a 4-alpha-glucanotransferase (GTase).

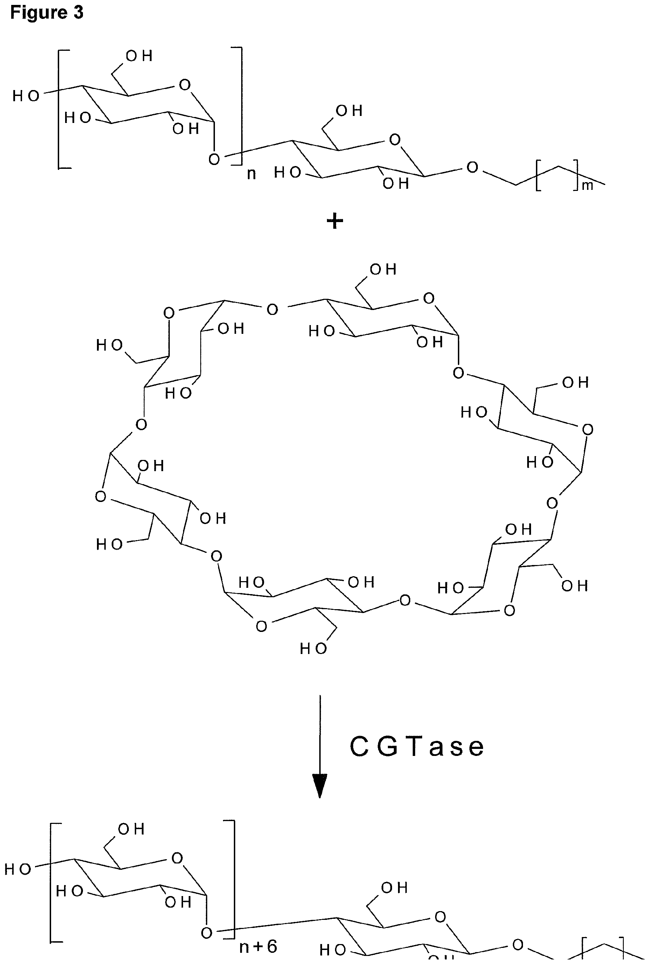

Figure 3 is a schematic view of the CGTase catalysed coupling between an alkyl glycoside and α-cyclodextrin. The hydrocarbon chain can be of varying length (m>3) and the carbohydrate part of the acceptor can be of varying length, with n typically being 0, 1 or 2. The coupling product contains 6 additional glucose residues.

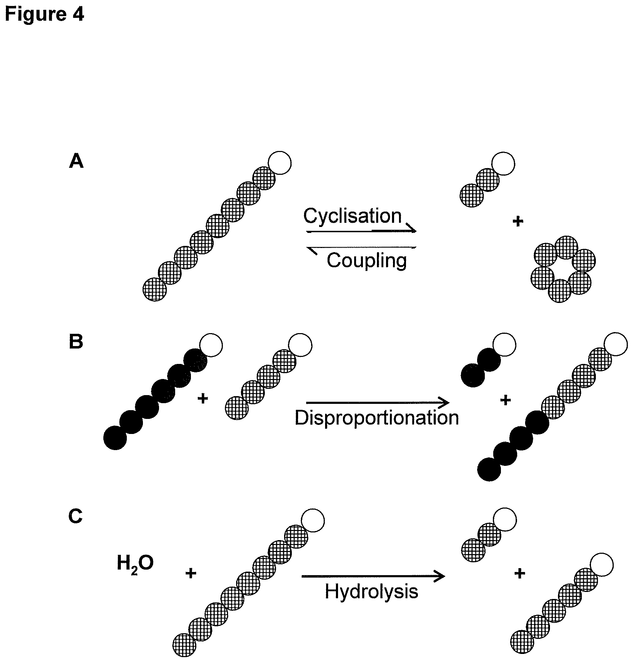

Figure 4 is a schematic view of CGTase and GTase catalysed reactions. White circles represent reducing end glucose residues while other glucose residues are shown as chequered or black circles. Figure 4A shows an example of the cyclisation and coupling reactions using alpha-cyclodextrin, but the cyclic alpha-glucan molecules vary in size dependent on the enzyme used. Figure 4B shows the disproportionation reaction and 4C the hydrolysis reaction. The size of the noncyclic substrates can vary from short oligosaccharides to long (or even branched) polysaccharides.

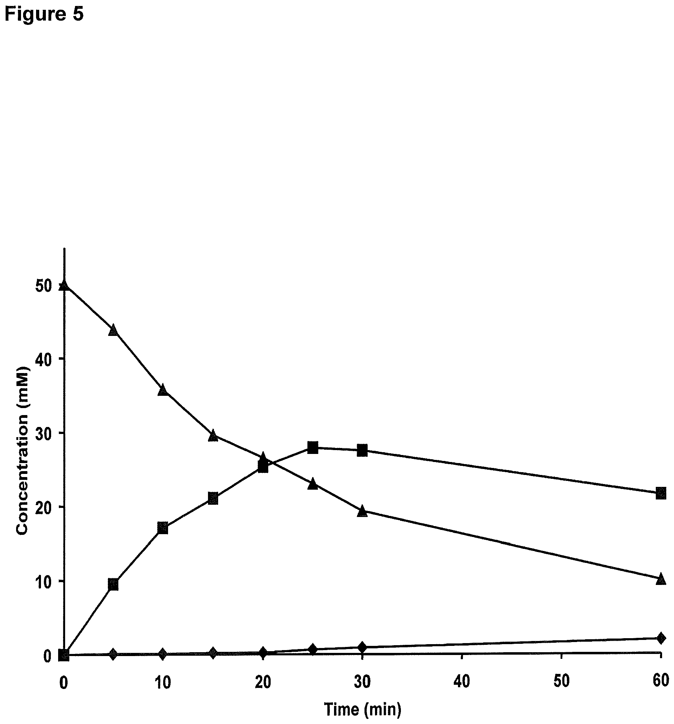

Figure 5 shows the time course of the CGTase catalysed reaction between an excess of α-cyclodextrin and dodecylmaltoside (filled triangles), yielding primary coupling product (filled squares) and secondary coupling product (filled diamonds).

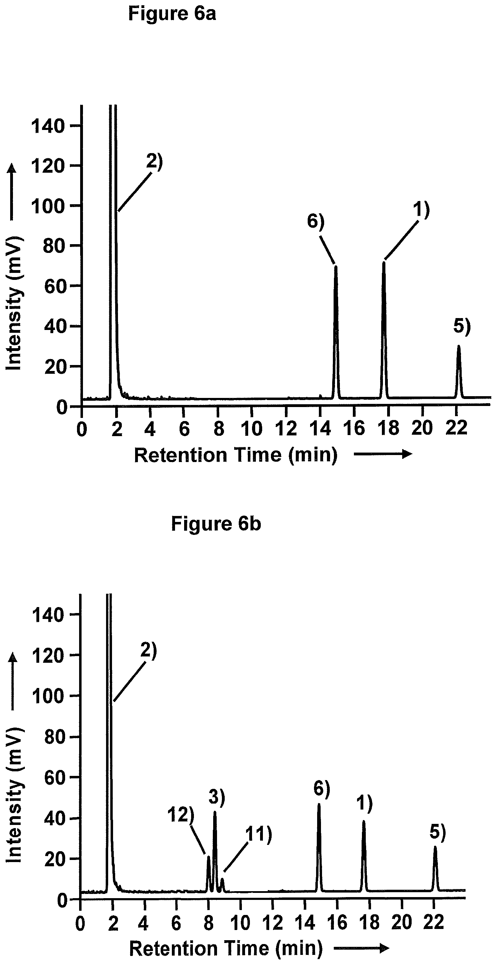

Figure 6 depicts the HPLC chromatograms before (a) and after 10 minutes (b) of a CGTase catalysed reaction between α-cyclodextrin (2) and an equimolar mixture of dodecyl-β-D-glucoside (5), dodecyl-β-maltoside (1) and dodecyl-β-maltotrioside (6).
All the primary coupling products (dodecyl-β-maltoheptaoside (11), dodecyl-β-maltooctaoside (3) and dodecyl-β-maltononaoside (12)) were formed.

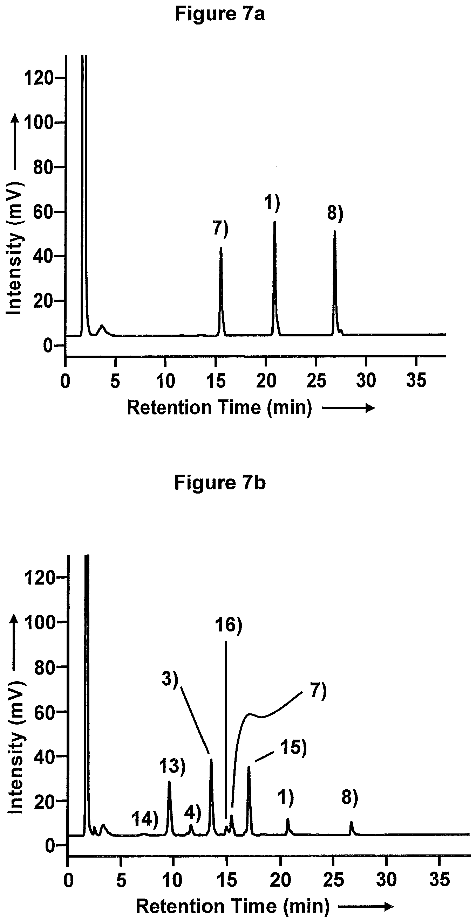

Figure 7 depicts the HPLC chromatograms before (a) and after 10 minutes (b) of a CGTase catalysed reaction between α-cyclodextrin and an equimolar mixture of decyl-β-maltoside (7), dodecyl-β-maltoside (1) and tetradecyl-β-maltoside (8).
Primary coupling products (alkyl-maltooctaosides: 13, 3, 15) were the main products and small amounts of secondary coupling products (alkyl maltotetradecaosides: 14, 4, 16) were formed as well.

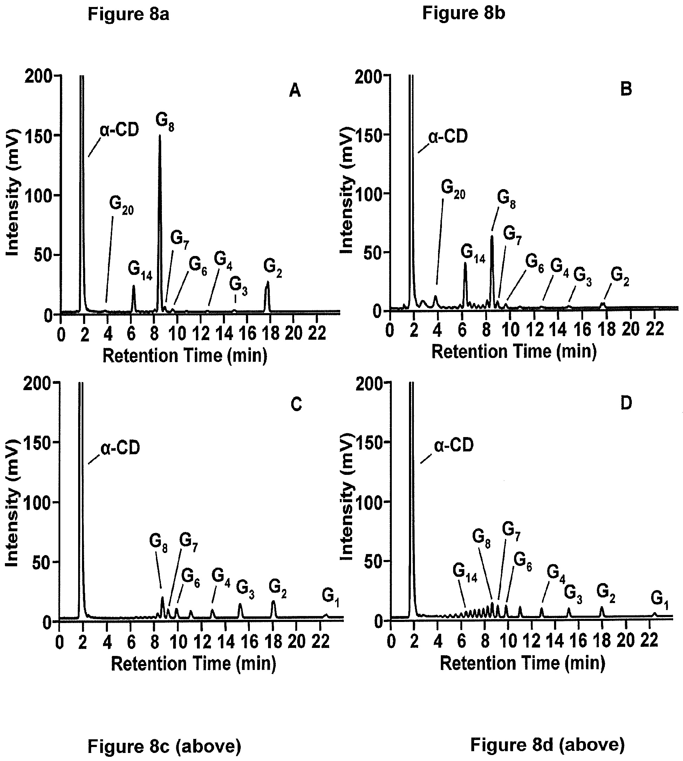

Figure 8 shows a comparison of two different CGTases: HPLC chromatogram after 25 min (A, Figure 8a) and 1 h (B, Figure 8b) reaction using CGTase from *Bacillus macerans* (50 mM DDM; 400 mM α-CD; pH 5.5; 60 °C). HPLC chromatogram after 25 min (C, Figure 8c) and 1 h (D, Figure 8d) reaction using CGTase from *Thermoanaerobacter* (50 mM DDM; 400 mM α-CD; pH 5.5; 60 °C). Gₙ means the dodecyl glycosides having n glucose residues.

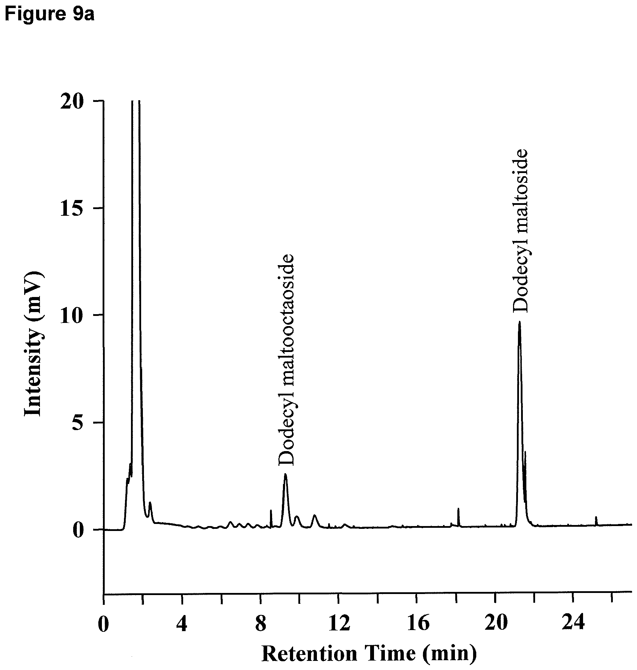

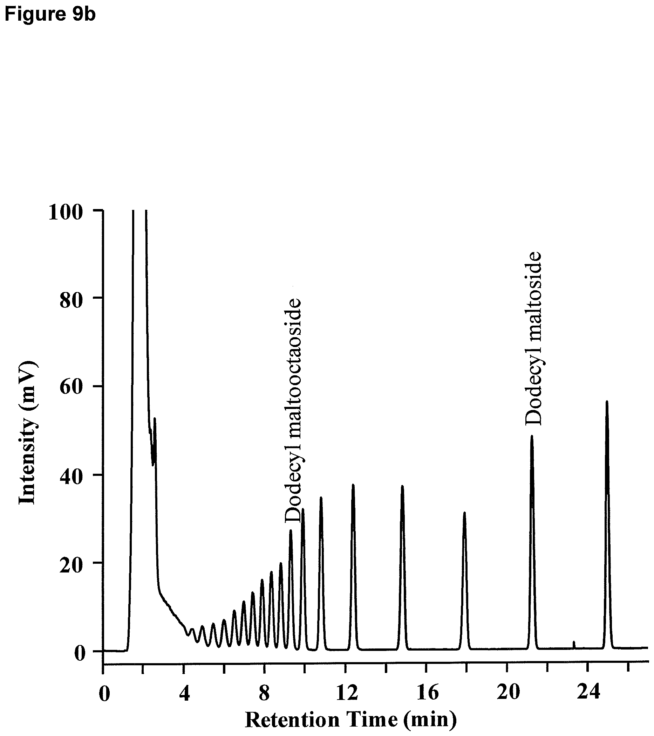

Figure 9 depicts a HPLC chromatogram after 20 minutes (a) and 5 h (b) of a reaction between starch and dodecyl-β-maltoside using *Bacillus macerans* CGTase as catalyst.

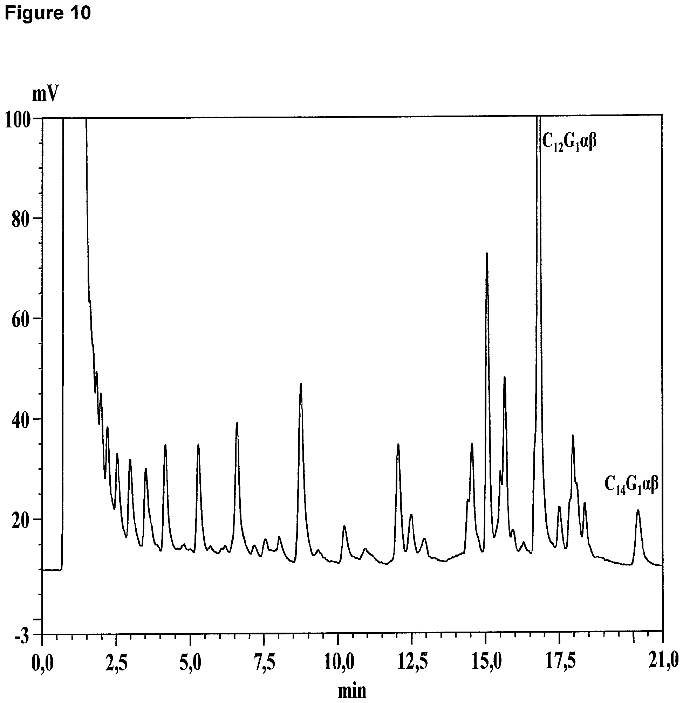

Figure 10 shows a reversed phase HPLC chromatogram of a reaction mixture from the reaction between APG 600 UP and soluble starch catalyzed by CGTase from *Bacillus macerans.* The main components in the starting material, dodecyl-D-glucoside (α and β isomers; C12G1αβ) and tetradecyl-D-glucoside (α and β isomers; C14G1αβ), are indicated. In addition, the reaction mixture contains a range of products formed by elongation of these starting materials with varying number of glucose residues originating from the soluble starch.

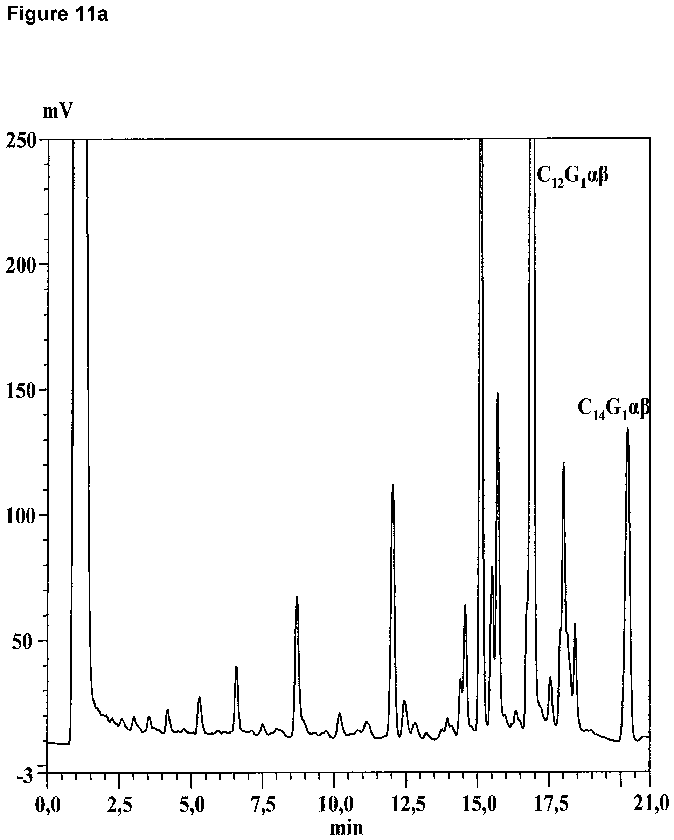

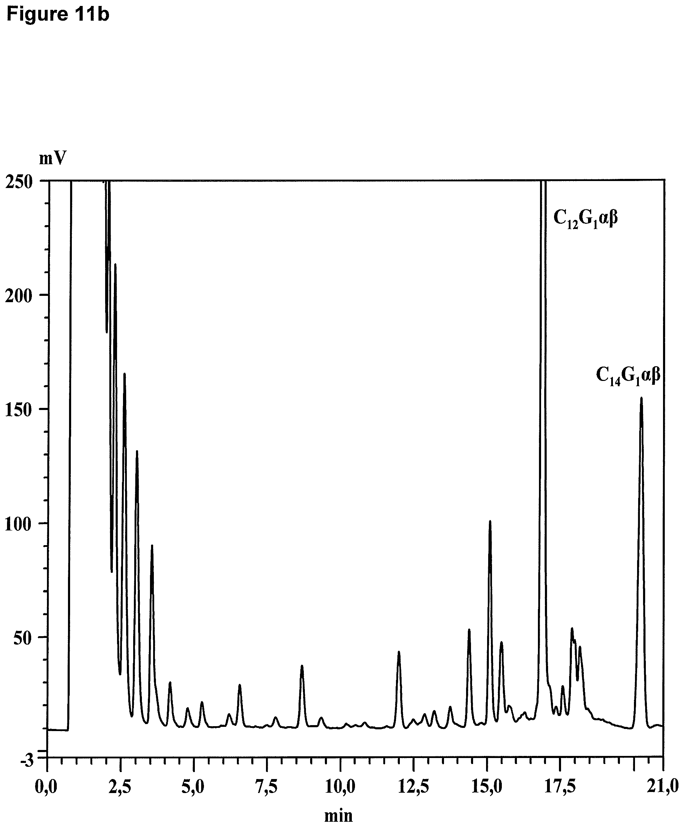

Figure 11 shows a reversed phase HPLC chromatogram of a reaction mixture from the reaction between APG 600 UP and soluble starch catalyzed by CGTase from *Thermoanaerobacter* (Figure 11a) or by archaeal *Thermococcus-like* GTase (Figure 11b). The main components in the starting material, dodecyl-D-glucoside (α and β isomers; C12G1αβ) and tetradecyl-D-glucoside (α and β isomers; C14G1αβ), are indicated. In addition, the reaction mixture contains a range of products formed by elongation of these starting materials with varying number of glucose residues originating from the soluble starch.

### DETAILED DESCRIPTION OF THE INVENTION AND

### PREFERRED EMBODIMENTS THEREOF

The invention is a method to synthesize alkyl glycosides having oligomeric head groups, and in particular, those having both long hydrophobic and hydrophilic groups. The properties of a surfactant depend very much on the balance between its hydrophilic and hydrophobic parts. Concerning alkyl glycosides, this means that when for some reason it is desired to use a long hydrophobic group, then the hydrophilic groups preferably should be long as well, thus being oligomeric with respect to monosaccharide residues. For surfactants coming into contact with humans, long hydrophobic groups are often desired because of their lower tendency to cause irritation.

In summary, the invention herein is a process for the preparation of long-chain alkyl glycosides by enzymatic synthesis, comprising the steps of providing a glycosyl donor; providing a glycosyl acceptor; and allowing the glycosyl donor and glycosyl acceptor to react with each other in the presence of an selected from the group of cyclomaltodextrin glucanotransferase (EC number 2.4.1.19) and 4-alpha-glucanotranserase, (EC number 2.4.1.25) to produce (an) alkyl glycoside(s) having an elongated carbohydrate part.

Here, a novel method to elongate the carbohydrate part of alkyl glycosides is presented. Several monosaccharide residues, from 2 to 26, e.g. from 4 to 26, and preferably at least 6, such as 7, 8 or 9 residues, depending on the glycosyl donor used, can be added in a single step using an enzyme as catalyst and without any need for protection of the substrate molecules (Figures 2-3). Due to the mild reaction conditions and the highly selective enzymatic catalyst, unwanted side reactions occur only to a very low extent. Furthermore, only small amounts of waste are produced. Subsequent to the first trans-glycosylation reaction, the enzyme can catalyze secondary trans-glycosylation reactions between different alkyl glycosides in the reaction mixture. These reactions are called disproportionation reactions and cause the formation of a spectrum of alkyl glycosides with varying length of the carbohydrate chain. The aforementioned enzymatic reactions can be stopped by inactivation of the enzyme by heat or addition of acid, base or other agents or by removal of the enzyme. For some applications, the reaction mixtures can be useful without further processing, but in most cases partial or complete purification of a single product or a range of products from the reaction mixture will be carried out. The purification can be achieved by a variety of methods including chromatography, extraction, precipitation, filtration and centrifugation as known in the art.

### The glycosyl donor

Preferably the glycosyl donor is a cyclic, linear or branched oligo- or polysaccharide or a mixture of such compounds. In the invention, at least 2 and maximum 26, e.g. from 4 to 26, and preferably at least 6, such as 7, 8 or 9, monosaccharide residues are transferred in a single step from the glycosyl donor to the glycosyl acceptor. The glycosyl donor has the general formula Gn, wherein G is derived from a reducing saccharide having 5 or 6 carbon atoms, i.e. G is a monosaccharide residue having 5 or 6 carbon atoms, e.g. glucose, fructose or hexose, and n is the number of monosaccharide residues in the glycosyl donor and is at least 2. In some cases the glycosyl donor can have up to several hundreds of monosaccharide residues for example in polysaccharides such as starches.

The glycosyl donor preferably comprises a compound selected from the group consisting of cyclic carbohydrate, i.e. a carbohydrate in which the chain of monosaccharide residues forms a closed loop (such as α-, β-, γ-cyclodextrin or larger cyclic alpha-glucans), linear oligosaccharides and polysaccharides, such as starch, hemicellulose, lactose, maltose, etc. The invention works with different glycosyl donors. When cyclodextrins are used as glycosyl donors very high yields of coupling products can be obtained. However, oligosaccharides and polysaccharides, such as starch, can be used as glycosyl donors as well, which is important for lowering the production costs. Also in these cases, coupling products are obtained since the enzyme can synthesize cyclodextrins from linear saccharides. In addition, disproportionation reactions may occur. The glycosyl donor may consist of a mixture of cyclic carbohydrates, linear or branched oligo- or polysaccharides. Generally however, preferably only one type of compound is used as the glycosyl donor, since it may result in a higher yield, without a need for further purification of the different elongated alkyl glycosides produced.

### The glycosyl acceptor

In the invention, the glycosyl acceptor is preferably an alkyl glycoside or a mixture of alkyl glycosides. The acceptor has a hydrocarbon chain containing at least 7 carbon atoms for better results. With less than 7-8 carbon atoms low yield of products is obtained and the reaction is not efficient, see Ref. 10.

The acceptor in the context of the present invention should correspond to the general formula Gₘ-OR, wherein m is the number of monosaccharides residues of the acceptor; m is at least 1 and may suitably be up to 20, but could also be a larger number of monosaccharide residues. R is the alkyl group or monovalent radical containing at least 7 carbon atoms and preferably not more than 20, although longer alkyl glycosides might also be suitable for the present invention.

Useful acceptors include alkyl glucosides, alkyl maltosides and alkyl polyglycosides which are complex mixtures of alkyl glycosides, differing with respect to the number of monosaccharide residues, the bonds between those and the length of the hydrocarbon chain. In general preferred acceptors are those with linear or branched, saturated or unsaturated alkyl groups (R) with 8, 10, 12, 14, 16 and 18 carbon atoms and containing, for example, up to 3 double bonds.

Anomerically pure alkyl glycosides for use as acceptors in the present invention are commercially available or can be synthesized using enzymatic methods. Octyl-β-D-glucoside can thus be prepared by direct condensation between glucose and octanol ^{[11]} and by transglycosylation from p-nitrophenyl-β-D-glucopyranoside and octanol [12]

### The enzyme

The enzyme used as catalyst in the invention is able to transfer at least two monosaccharide residues at a time from the glycosyl donor to the glycosyl acceptor, which should be an alkyl glycoside or a mixture of alkyl glycosides. The enzyme is selected from the group of cyclomaltodextrin glucanotransferase (EC number 2.4.1.19) and 4-alpha-glucanotranserase, (EC number 2.4.1.25), being able to transfer at least two monosaccharide residues at a time from the glycosyl donor to the glycosyl acceptor . In addition to hydrolysis reactions, glycoside hydrolase enzymes can often catalyse various types of transglycosylation reactions. Many enzymes suitable for the invention belong to the α-amylase family (glycoside hydrolase family 13) ^{[8]}, but glycoside hydrolases from other families (such as glycoside hydrolase family 57) catalysing the reactions described here can be used as well. The enzyme used in the present invention is a cyclomaltodextrin glucanotransferase (EC number 2.4.1.19) ((1-4)-alpha-D-glucan: (1-4)-alpha-D-glucan 4-alpha-D[(1-4)-alpha-D-glucano]-transferase; CGTase). This type of enzyme is sometimes called cyclodextrin glycosyltransferase, cyclodextrin glucanotransferase or cyclodextrin glucanyltransferase (also abbreviated CGTase). The most well-known reaction of CGTases is the production of cyclodextrins from starch (Figure 4). However, they can also use cyclodextrins as substrates and add them to suitable acceptors having a carbohydrate nature. This particular reaction is called coupling reaction. Furthermore, CGTases can transfer oligosaccharides from a linear oligosaccharide to an acceptor (disproportionation reaction) and to a limited extent also hydrolyse cyclodextrins and linear substrates. The coupling reaction normally uses pure carbohydrates as acceptors, with elongation of maltose to maltooctaose, maltotetradecaose etc. as a typical example ^{[9]}. However, it has been shown that also some derivatised carbohydrates function as acceptors in CGTase catalysed coupling reactions. Recently, the coupling reactions between α-cyclodextrin and methyl-D-glucopyranosides (α and β) and phenyl-D-glucopyranosides (α and β) were optimized. However, the yields of the primary coupling products, methyl maltoheptaosides, were below 5 % and for the phenyl maltoheptaosides below 2 % ^{[10]}.

The cyclodextrin glucanotransferase (CGTase) from *Bacillus stearothermophilus* has been used for glycosylation of surfactants, but it does not produce coupling products like those produced in the present invention ^{[6, 7]}. Using this enzyme with sucrose laurate (a sugar ester) as acceptor, products with 1-3 additional glucose residues were formed ^{[6]}. Also alkyl maltosides have been used as acceptors in glycosylation reactions catalyzed by this enzyme with dextrin as glycosyl donor ^{[7]}. Only one or two glucose residues were added to the acceptors thus producing products with maximum 4 glucose residues, which shows that no coupling reaction occurred.

CGTases have previously not been used to elongate the carbohydrate part of alkyl glycosides having long hydrocarbon chains (i.e. >6 carbon atoms) by transfer of more than 3 glucose residues.

An example of a particularly useful enzyme for the present invention is *Bacillus macerans* CGTase commercially available from Amano Enzyme Europe, U.K. This enzyme catalyzes the coupling reaction between α-cyclodextrin and a variety of acceptors. For special combinations of acceptor and donor substrates, other enzymes are better suited. When a broader product distribution (a range of products varying in carbohydrate chain length) is desired an enzyme with high disproportionation activity is beneficial. The *Thermoanaerobacter* CGTase (Toruzyme® 3.0 L) from Novozymes A/S, Denmark is an example of such an enzyme.

The invention herein is also carried out using glycoside hydrolases classified under family 57, classified as 4-alpha-glucanotranserase, E.C. number 2.4.1.25; systematic name: (1-4)-alpha-D-glucan: (1-4)-alpha-D-glucan 4-alpha-D-glycosyl tranferase; GTase), catalyse similar reactions (although with cyclic alpha-glucans of larger ring size than traditional cyclodextrins, which have 6-8 glucose residues) and are suitable catalysts. They are also capable of transferring at least 6-9 glucose residues at a time from the glycosyl donor to the glycosyl acceptor and up to 26 residues. The archaeal *Thermococcus-like* GTase with a sequence deposited under GeneBank accession number EU138451, is an example of such an enzyme.

Enzymes generally convert substrate molecules dissolved in the reaction medium and the concentration of dissolved substrate therefore determines the rate of enzymatic conversion. When surfactants are dissolved in water, they are dissolved as separate molecules up to a concentration called "critical micelle concentration" (CMC). When the overall substrate concentration exceeds CMC, the concentration of separately dissolved surfactant molecules does not increase further. Instead, additional surfactant molecules form micelles and in this form enzymes normally do not convert them. Octyl-α-D-glucopyranoside has a CMC of 17-19 mM in water ^{[13]}, which makes it reasonable to expect that it could be efficiently converted enzymatically. However, dodecyl-P-D-glucoside and dodecyl-β-maltoside have CMCs more than two orders of magnitude lower (0.11-0.13 mM for the glucoside and 0.12-0.14 for the maltoside ^{[13]}), and therefore are not expected to be easily elongated in such an enzymatic reaction, which makes it highly surprising that they actually can be elongated enzymatically in aqueous solution. Even more surprising is that tetradecyl-β-maltoside, hexadecyl-β-maltoside and oleyl-β-maltoside, which can be predicted to have even lower CMC, can be elongated as well as shown in Example 1 and 2.

In order to optimize the yield of a certain product according to the invention, it is important to stop the enzymatic reactions at a suitable stage. This can be done by inactivation of the enzyme or by removal of it. A particularly attractive way to separate the enzyme from the product is to use the enzyme in the immobilized form, typically covalently bound to a solid support material. The immobilized enzyme can easily be filtered off after reaction in a batch reactor, or it can be used in a packed bed reactor through which the reactant solution is passed. In the latter case, the residence time in the reactor is adjusted to achieve a suitable conversion in the enzymatic reaction.

Another advantage of the present invention is that it does not generate anomer mixtures. The glycosidic linkage formed between the acceptor and the added carbohydrate part will be either α or β depending on the enzyme used. CGTases for example give α bonds. The anomer distribution of the acceptor will be reflected in the product, although it might be changed somewhat due to the acceptor specificity of the enzyme. It is an important advantage that the invention gives anomerically pure products, when the acceptor is anomerically pure. Anomerically pure products cannot be obtained by previously described synthesis methods using acid catalysts, even with anomerically pure starting materials ^{[2]}.

### EXAMPLE 1

### Materials

*Enzymes. Bacillus macerans* CGTase (EC. 2.4.1.19) was purchased from Amano Enzyme Europe Ltd.(Milton Keynes, U.K.) and *Thermoanaerobacter* CGTase (Toruzyme® 3.0 L) was from Novozymes A/S (Bagsvaerd, Denmark).

*Chemicals.* Dodecyl-β-maltoside (DDM) was obtained from Anatrace Inc., Maumee Ohio, USA, alpha-cyclodextrin (α - CD) was from Wacker Chemie AG (Stuttgart, Germany) and other chemicals were of pro-analysis grade from VWR International.

*Synthesis of dodecyl-β-maltooctaoside (DDMO).* The substrates (50 mM DDM and 400 mM α - CD) were dissolved in 8 ml 10 mM Na-citrate buffer, pH 5.5, in a 100 ml round-bottom flask. The flask was placed in a temperature controlled water bath at 60°C, and the reaction was initiated by adding 40 µl of *Bacillus macerans* CGTase (final concentration 0.0219 g·L⁻¹). The reaction was terminated after 25 minutes reaction time by heat treatment in boiling water for 2 minutes. The mixture was then directly diluted with 60 ml methanol:water (20:80) solution and applied onto C-18 RP flash column preconditioned with methanol:water (20:80) solution. Thereafter, the carbohydrates were eluted with methanol:water (20:80) solution. DDMO was then eluted by increasing the methanol content to 75% v/v. From those fractions, the solvent (methanol : water 75:25) was evaporated in a rotary evaporator at 45 °C after which the solids were dispersed in 50 ml methanol to further extract moisture from the preparation. Finally, the product was recovered by evaporating the solvent followed by precipitation using 30 ml acetone. The white DDMO crystals were isolated and placed to dry in a vacuum desiccator equipped with molecular sieves (3Å). The product purity was estimated to be approx. 86 % by HPLC. The overall yield based on the weight of the substrate DDM and the product was 54 % mol DDMO / mol DDM. The time course during 60 minutes of a reaction between α-cyclodextrin and DDM is shown in Figure 5. The secondary coupling product starts to appear after 25 minutes reaction time.

*HPLC analysis.* Samples collected during the development work were analysed using a HPLC system from Merck Hitachi (Lachrom (pump L-7100, interface L-7000, Autosampler L-7250 with a 20 µl injection loop), Hitachi, Ltd. Tokyo, Japan) provided with a C-18 RP column (Kromasil 100-5C18, L: 25 cm, i.d.: 4.6 mm, Eka Chemicals AB, Separation Products, Bohus, Sweden) connected to an Evaporative Light Scattering Detector (ELSD) (Alltech 500 ELSD, Alltech Associates, Inc., Deerfield, USA) operating at 99 °C with a nebuliser gas flow of 2.73 SLPM (Standard Litres Per Minute). Elution (1 ml/min) involved a linear gradient of 35:65 to 60:40 acetonitrile:water (with 0.1 % v/v acetic acid) during 23 minutes with a final hold time of 1 minute at 60:40 acetonitrile : water. Standard curves were made for DDM and DDMO.

### EXAMPLE 2

### Other acceptors

The coupling reaction with α-cyclodextrin as glycosyl donor described in EXAMPLE 1 works with different acceptor substrates. Using decyl -β-maltoside, tetradecyl-β-maltoside, hexadecyl-β-maltoside or oleyl-β-maltoside as acceptors thus yields decyl-β-maltooctaoside, tetradecyl-β-maltooctaoside, hexadecyl-β-maltooctaoside and olcyl-β-maltooctaoside as primary coupling products under the conditions described in EXAMPLE 1 (Figure 6). Likewise dodecyl-β-D-glucoside and dodecyl-β-maltotrioside work as substrates yielding dodecyl-β-maltoheptaoside and dodecyl-β-maltononaoside as primary coupling products (Figure 7). Furthermore, alkyl-α-glycosides can be elongated in the same way as the β-isomers. In addition, complex mixtures of alkyl glycosides, such as commercially available alkyl polyglycosides, can be used as acceptor substrates in the coupling reactions with α-cyclodextrin as glycosyl donor, yielding the corresponding complex product mixtures in which the carbohydrate part has been elongated with 6 glucose residues. The results show that the method works well for the elongation of the carbohydrate group of a wide range of alkyl glycosides.

### EXAMPLE 3

### Multiple coupling products

Provided that an excess of α-cyclodextrin is used as glycosyl donor, the reactions described in EXAMPLES 1 and 2 can be continued to yield secondary and further coupling products. In each step the carbohydrate chain is elongated with 6 glucose residues. Secondary coupling products are seen in Figure 7. The results show that multiple additions of the glucose residues from α-cyclodextrin to alkyl glycosides are possible. This makes it possible to synthesize alkyl glycosides with very long carbohydrate chains under good control, although there will be a distribution of products which have gone through varying numbers of coupling steps.

### EXAMPLE 4

Other cyclodextrins and cyclic alpha-glucans of different ring sizes.

The reactions described in EXAMPLES 1-3 can be carried out using different cyclodextrins as glycosyl donors. When β-cyclodextrin is used as glycosyl donor, each coupling step adds 7 glucose residues to the carbohydrate chain. When γ-cyclodextrin is used as glycosyl donor, each coupling step adds 8 glucose residues to the carbohydrate chain. These results show that it is possible not only to add multiples of 6 glucose residues, but also multiples of 7 and 8 residues to the carbohydrate part of the acceptor (Figure 6). By a proper combination of the length of the carbohydrate part of the acceptor, the type of cyclodextrin used and the number of coupling steps, it is thus possible to synthesize products with any length of the carbohydrate chain. In addition, GTascs can add large cyclic alpha-glucans, which makes it possible to achieve an elongation with up to at least 26 monosaccharide residues in a single step.

### EXAMPLE 5

### Broadening of the product pattern by disproportionation reactions

The relative activity of different CGTases in coupling and disproportionation reactions varies. By choosing an enzyme with a relatively high disproportionation activity and/or by prolongation of the reaction time, a high amount of disproportionation products can be obtained. These products differ only concerning the number of glucose residues it contains. An example of a CGTase giving a high amount of disproportionation reactions at an early stage is the one from *Thermoanaerobacter* (Figure 8). These products are attractive for applications when mixtures of closely related surfactants are effective.

### EXAMPLE 6

### Starch as glycosyl donor

The reactions described in EXAMPLES 1-2 can be carried out using oligosaccharides or starch as glycosyl donor. In these reactions, cyclodextrins are formed as intermediate products. After a short reaction time, coupling products dominate and after a longer reaction time increasing amounts of disproportionation products are formed (Figure 9). Since starch is considerably cheaper than cyclodextrins this opens the possibility for the production of cheaper products.

### EXAMPLE 7

### Alkyl polyglycosides as acceptor and starch as glycosyl donor

The reactions described in EXAMPLES 1-2 and 6 can be combined and carried out using alkyl polyglycosides as acceptor and oligosaccharides or starch as glycosyl donor. As in EXAMPLE 6, cyclodextrins are initially formed as intermediate products. Therefore after a short reaction time, coupling products dominate and thereafter increasing amounts of disproportionation products are formed (Figure 10). *Elongation of APG 600 UP (APG).* APG 600 UP (APG) was obtained from Cognis GmbH (Düsseldorf, Germany), soluble starch was BDH Chemicals Ltd. (Poole, England), and other chemicals were of pro-analysis grade from VWR International (West Chester, PA, USA). Soluble starch (250g/l) was dissolved in 20 ml 10 mM Na-citratc buffer, pH 6, in a 100 ml round-bottom flask. The flask was placed in a temperature controlled water bath at 60°C, and the reaction was initiated by adding 100 µl of *Bacillus macerans* CGTase (final concentration of 0.0438 g·L⁻¹). After 90 minutes, the acceptor (APG, 25 g/l) was added to the reaction mixture. Fresh enzyme (100 µl each time) was added 30 and 90 minutes after the acceptor was added. The reaction was terminated after 4 hours' reaction time by heat treatment in boiling water for 5 minutes. The mixture was then directly diluted with methanol:water (20:80) solution and applied onto C-18 RP flash column preconditioned with methanol:water (20:80) solution. Thereafter, the carbohydrates were eluted with methanol:water (20:80) solution. Modified APG was then eluted by 100% v/v methanol. From this fraction, the solvent was evaporated in a rotary evaporator at 45 to 55 °C after which the solids were dispersed in 50 ml methanol to further extract moisture from the preparation. Finally, the product was recovered by evaporating the solvent followed by precipitation using 30 ml acetone. The white product crystals were isolated and placed to dry in a vacuum desiccator equipped with molecular sieves (3Å).

### EXAMPLE 8

### Use of different enzymes

Different enzymes can be used as catalysts in this invention. The reaction described in EXAMPLE 7 was thus catalyzed by the CGTase from *Thermoanaerobacter* (see EXAMPLE 1) or the archaeal *Thermococcus-like* GTase, which was cloned for expression in our laboratory. Both enzymes catalyzed the elongation of the carbohydrate part of the alkyl polyglycoside APG 600 UP. The proportions of different elongation products were different depending on which enzyme was used. The *Thermoanaerobacter* enzyme produced primarily products eluting rather late in the reversed phase chromatogram (Figure 11a), thus representing products with moderate elongation of the carbohydrate chain, while the archaeal *Thermococcus-*like GTase produced several alkyl glycoside products eluting quite early in the chromatogram (Figure 11b) and thus having very long carbohydrate parts.

### EXAMPLE 9

### Immobilized enzyme

In order to easily separate enzyme and product, the enzyme can be immobilized on a suitable support material prior to use in the invention. This also facilitates re-use of the enzyme, thereby reducing the enzyme cost per kg of product. A typical immobilization method, which has been successfully used with the *Bacillus macerans* CGTase is covalent coupling to commercially available Eupergit C.

As to the manner of usage and operation of the present invention, the same should be apparent from the above description. Accordingly, no further discussion relating to the manner of usage and operation will be provided.

### REFERENCES

[1] W. von Rybinsky, K. Hill, Angew. Chem. Int. Ed. 1998, 37, 1328.
[2] K. Katsuraya, T. Shibuya, K. Inazawa, H. Nakashima, N. Yamamoto, T. Uryu, Macromolecules 1995, 28, 6697.
[3] T. Shoji, N. Ikushima, N. Takahashi, K. Katsuraya, H. Nishibashi, T. Uryu, T. Yoshida, N. Yamamoto, H. Nakajima, S. Shigeta, (Dainippon Ink & Chemicals, Japan). Application: JP, 1993, p. 31 pp.
[4] C. Panintrarux, S. Adachi, Y. Araki, Y. Kimura, R. Matsuno, Enzyme Microb. Technol. 1995, 17, 32.
[5] G. Richard, S. Morel, R.-M. Willemot, P. Monsan, M. Remaud-Simeon, Carbohydrate Research 2003, 338, 855.
[6] K. Okada, H. Zhao, M. Izumi, S. Nakajima, N. Baba, Biosci., Biotechnol., Biochem. 2007, 71, 826.
[7] H. Zhao, H. Naito, Y. Ueda, K. Okada, K. Sadagane, M. Izumi, S.
[8] B. Henrissat, Biochemical Journal 1991, 280, 309.
[9] D. Vetter, W. Thorn, Starch/Stärke 1992, 44, 229.
[10] S. H. Yoon, J. F. Robyt, Carbohydrate Research 2006, 341, 210. Nakajima, N. Baba, Biosci., Biotechnol., Biochem. 2008, 72, 3006.
[11] G. Ljunger, P. Adlercreutz, B. Mattiasson, Enzyme Microb. Technol. 1994, 16, 751.
[12] P. Turner, D. Svensson, P. Adlercreutz, E. N. Karlsson, Journal of Biotechnology 2007, 130, 67.
[13] I. Ribosa, J. SanchezLeal, F. Comelles, M. T. Garcia, Journal of Colloid and Interface Science 1997, 187, 443.

## Claims

1. A process for the elongation of (an) alkyl glycoside(s) by enzymatic synthesis, comprising:
a. providing a glycosyl donor having at least 2 monosaccharide residues and comprising a compound selected from the group consisting of cyclic carbohydrates, linear oligosaccharides and polysaccharides;
b. providing an alkyl glycoside containing at least 7 carbon atoms in the alkyl chain as a glycosyl acceptor;
c. providing an enzyme capable of catalyzing the transfer of at least 2 monosaccharide residues at a time from the glycosyl donor to the glycosyl acceptor, the enzyme being selected from the group consisting of cyclomaltodextrin glucanotransferase, EC number 2.4.1.19, and 4-alpha-glucanotranferase, EC number 2.4.1.25; and
d. allowing the glycosyl donor and glycosyl acceptor to react with each other in the presence of the enzyme to produce an alkyl glycoside having an elongated carbohydrate part.

2. The process according to claim 1, wherein said enzyme is selected from the group cyclomaltodextrin glucanotransferase, EC number 2.4.1.19.

3. The process according to claim 2, wherein said cyclomaltodextrin glucanotransferase is from *Bacillus macerans* or *Thermoanaerobacter.*

4. The process according to claim 1, wherein said 4-alpha-glucanotranferase is from *Thermococcus sp.*

5. The process according to any of claims 1-4, wherein the enzyme used is in an immobilized form, preferably covalently bound to a solid support material.

6. The process according to any of claims 1-5, wherein the alkyl glycoside(s) contains at most 20 carbon atoms in the alkyl chain.

7. The process according to any of claims 1-6, wherein the alkyl glycoside(s) contains 8, 10, 12, 14, 16 or 18 carbon atoms in the alkyl chain, preferably the alkyl groups (R) are linear or branched, saturated or unsaturated.

8. The process according to any of claims 1-7, wherein the alkyl glycosides are selected from the group consisting of alkyl glucosides, alkyl maltosides and alkyl polyglycosides, preferably from the group consisting of alkyl glucosides and alkyl polyglycosides.

9. The process according to any of claims 1-8, wherein a mixture of alkyl glycosides is used.

10. The process according to any of the preceding claims, wherein the glycosyl donor is selected from the group consisting of cyclodextrin, maltodextrins, starch and partially degraded starch; preferably selected from the group consisting of a-cyclodextrin, maltodextrins, starch and partially degraded starch; preferably selected from the group consisting of maltodextrins, starch and partially degraded starch.

11. The process according to any of the preceding claims, wherein the glycosyl donor provides up to 26 monosaccharide residues to the alkyl glycoside.

12. The process according to claim 11, wherein the glycosyl donor provides 2-26 monosaccharide residues to the alkyl glycoside, preferably 4-26 monosaccharide residues to the alkyl glycoside.

13. The process according to claim 11, wherein the glycosyl donor provides at least 6 monosaccharide residues to the alkyl glycoside.

14. The process according to claim 11, wherein the glycosyl donor provides 7, 8 or 9 monosaccharide residues to the alkyl glycoside.

15. The process according to any of the preceding claims, wherein the alkyl glycoside having an elongated carbohydrate part obtained has at least 7 carbon atoms in the alkyl chain and head groups thereon contain at least 3 monosaccharide residues.

## Patentansprüche

1. Verfahren zur Verlängerung eines oder mehrerer Alkylglykoside durch enzymatische Synthese, umfassend:
a. die Bereitstellung eines Glykosyldonators mit mindestens 2 Monosaccharidresten und umfassend eine aus der aus cyclischen Kohlenhydraten, geradkettigen Oligosacchariden und Polysacchariden bestehenden Gruppe ausgewählte Verbindung,
b. die Bereitstellung eines Alkylglykosids mit mindestens 7 Kohlenstoffatomen in der Alkylkette als Glykosylakzeptor,
c. die Bereitstellung eines Enzyms, das dazu fähig ist, den gleichzeitigen Transfer von mindestens 2 Monosaccharidresten vom Glykosyldonator zum Glykosylakzeptor zu katalysieren, wobei das Enzym aus der aus Cyclomaltodextringlucanotransferase, EC-Nummer 2.4.1.19, und 4-alpha-Glucanotransferase, EC-Nummer 2.4.1.25, bestehenden Gruppe ausgewählt ist und
d. das Ermöglichen der Reaktion des Glykosyldonators mit dem Glykosylakzeptor in Gegenwart des Enzyms unter Bildung eines Alkylglykosids mit einem verlängerten Kohlenhydratteil.

2. Verfahren nach Anspruch 1, wobei das Enzym aus der Gruppe Cyclomaltodextringlucanotransferase, EC-Nummer 2.4.1.19, ausgewählt ist.

3. Verfahren nach Anspruch 2, wobei die Cyclomaltodextringlucanotransferase aus *Bacillus macerans* oder *Thermoanaerobacter* stammt.

4. Verfahren nach Anspruch 1, wobei die 4-alpha-Glucanotransferase aus *Thermococcus sp.* stammt.

5. Verfahren nach einem der Ansprüche 1-4, wobei das Enzym in einer immobilisierten Form, vorzugsweise kovalent an ein festes Trägermaterial gebunden, eingesetzt wird.

6. Verfahren nach einem der Ansprüche 1-5, wobei das/die Alkylglykosid(e) höchstens 20 Kohlenstoffatome in der Alkylkette enthält.

7. Verfahren nach einem der Ansprüche 1-6, wobei das/die Alkylglykosid(e) 8, 10, 12, 14, 16 oder 18 Kohlenstoffatome in der Alkylkette enthält, wobei die Alkylgruppen (R) geradkettig oder verzweigt und gesättigt oder ungesättigt sind.

8. Verfahren nach einem der Ansprüche 1-7, wobei die Alkylglykoside aus der aus Alkylglucosiden, Alkylmaltosiden und Alkylpolyglykosiden bestehenden Gruppe, vorzugsweise aus der aus Alkylglucosiden und Alkylpolyglykosiden bestehenden Gruppe, ausgewählt sind.

9. Verfahren nach einem der Ansprüche 1-8, wobei man eine Mischung von Alkylglykosiden verwendet.

10. Verfahren nach einem der vorhergehenden Ansprüche, wobei der Glykosyldonator aus der aus Cyclodextrin, Maltodextrinen, Stärke und teilweise abgebauter Stärke bestehenden Gruppe, vorzugsweise aus der aus α-Cyclodextrin, Maltodextrinen, Stärke und teilweise abgebauter Stärke bestehenden Gruppe, bevorzugt aus der aus Maltodextrinen, Stärke und teilweise abgebauter Stärke bestehenden Gruppe, ausgewählt ist.

11. Verfahren nach einem der vorhergehenden Ansprüche, wobei der Glykosyldonator bis zu 26 Monosaccharidreste für das Alkylglykosid bereitstellt.

12. Verfahren nach Anspruch 11, wobei der Glykosyldonator 2-26 Monosaccharidreste für das Alkylglykosid, vorzugsweise 4-26 Monosaccharidreste für das Alkylglykosid, bereitstellt.

13. Verfahren nach Anspruch 11, wobei der Glykosyldonator mindestens 6 Monosaccharidreste für das Alkylglykosid bereitstellt.

14. Verfahren nach Anspruch 11, wobei der Glykosyldonator 7, 8 oder 9 Monosaccharidreste für das Alkylglykosid bereitstellt.

15. Verfahren nach einem der vorhergehenden Ansprüche, wobei das erhaltene Alkylglykosid mit einem verlängerten Kohlenhydratteil mindestens 7 Kohlenstoffatome in der Alkylkette enthält und die Kopfgruppen daran mindestens 3 Monosaccharidreste enthalten.

## Revendications

1. Procédé pour allonger un ou plusieurs alkylglycosides par une synthèse enzymatique, comprenant :
a. la fourniture d'un donneur de glycosyle ayant au moins deux résidus monosaccharidiques et comprenant un composé choisi dans le groupe consistant en les hydrates de carbone cycliques, les oligosaccharides linéaires et les polysaccharides ;
b. la fourniture d'un alkylglycoside contenant au moins 7 atomes de carbone dans la chaîne alkyle, en tant qu'accepteur de glycosyle ;
c. la fourniture d'une enzyme capable de catalyser le transfert d'au moins 2 résidus monosaccharidiques à la fois, du donneur de glycosyle à l'accepteur de glycosyle, l'enzyme étant choisie dans le groupe consistant en la cyclomaltodextrine glucanotransférase, EC numéro 2.4.1.19 et la 4-alpha-glucanotransférase, EC numéro 2.4.1.25 ; et
d. le fait de permettre au donneur de glycosyle et à l'accepteur de glycosyle de réagir l'un avec l'autre en présence de l'enzyme pour produire un alkylglycoside ayant une partie hydrate de carbone allongée.

2. Procédé selon la revendication 1, dans lequel ladite enzyme est choisie dans le groupe de la cyclomaltodextrine glucanotransférase, EC numéro 2.4.1.19.

3. Procédé selon la revendication 2, dans lequel ladite cyclomaltodextrine glucanotransférase provient de *Bacillus macerans* ou de *Thermoanaerobacter.*

4. Procédé selon la revendication 1, dans lequel ladite 4-alpha-glucanotransférase provient de *Thermococcus sp.*

5. Procédé selon l'une quelconque des revendications 1 à 4, dans lequel l'enzyme utilisée se présente sous une forme immobilisée, de préférence liée par liaison covalente à un matériau support solide.

6. Procédé selon l'une quelconque des revendications 1 à 5, dans lequel le ou les alkylglycosides contiennent au plus 20 atomes de carbone dans la chaîne alkyle.

7. Procédé selon l'une quelconque des revendications 1 à 6, dans lequel le ou les alkylglycosides contiennent 8, 10, 12, 14, 16 ou 18 atomes de carbone dans la chaîne alkyle, de préférence les groupes alkyle (R) sont linéaires ou ramifiés, saturés ou insaturés.

8. Procédé selon l'une quelconque des revendications 1 à 7, dans lequel les alkylglycosides sont choisis dans le groupe consistant en les alkylglucosides, les alkylmaltosides et les alkylpolyglycosides, de préférence dans le groupe consistant en les alkylglucosides et les alkylpolyglycosides.

9. Procédé selon l'une quelconque des revendications 1 à 8, dans lequel on utilise un mélange d'alkylglycosides.

10. Procédé selon l'une quelconque des revendications précédentes, dans lequel le donneur de glucosyle est choisi dans le groupe consistant en la cyclodextrine, les maltodextrines, l'amidon et l'amidon partiellement dégradé ; de préférence choisi dans le groupe consistant en l'α-cyclodextrine, les maltodextrines, l'amidon et l'amidon partiellement dégradé ; de préférence choisi dans le groupe consistant en les maltodextrines, l'amidon et l'amidon partiellement dégradé.

11. Procédé selon l'une quelconque des revendications précédentes, dans lequel le donneur de glycosyle fournit jusqu'à 26 résidus monosaccharidiques à l'alkylglycoside.

12. Procédé selon la revendication 11, dans lequel le donneur de glycosyle fournit 2 à 26 résidus monosaccharidiques à l'alkylglycoside, de préférence 4 à 26 résidus monosaccharidiques à l'alkylglycoside.

13. Procédé selon la revendication 11, dans lequel le donneur de glycosyle fournit au moins 6 résidus monosaccharidiques à l'alkylglycoside.

14. Procédé selon la revendication 11, dans lequel le donneur de glycosyle fournit 7, 8 ou 9 résidus monosaccharidiques à l'alkylglycoside.

15. Procédé selon l'une quelconque des revendications précédentes, dans lequel l'alkylglycoside ayant une partie hydrate de carbone allongée tel qu'obtenu possède au moins 7 atomes de carbone dans la chaîne alkyle, et les groupes de tête se trouvant sur cette dernière contiennent au moins 3 résidus monosaccharidiques.
